# EUROPEAN PATENT APPLICATION

(11) **EP 0 934 755 A2**
(43) Date of publication of application: **11.08.1999**
(21) Application number: 99101621.3
(22) Date of filing: 03.02.1999
(51) Int. Cl.: A61M 25/00, A61M 25/10, A61L 29/00

(54) **Fiber reinforced balloon and catheter**

(30) Priority: 09.02.1998 US 20957
(71) Applicant: MEDTRONIC, INC., Minneapolis, Minnesota 55432-3576 (US)
(72) Inventor: Kramer, Hans W., Temecula, California 92592 (US); Wu, Show-Mean, San Diego, California 92129 (US); Chen, Ziyun, San Diego, California 92128 (US)
(74) Representative: Hughes, Andrea Michelle

(57) **Abstract**

A medical tubing 10, and method for manufacturing the tubing, involves combining a plurality of strengthening fibres 46a-c with a polymer matrix 48. For the method of the present invention, a thermoplastic polymer component 38 is blended with a plurality of liquid crystal polymer particles 40. This mix is heated to a temperature below approximately 270°C and extruded to establish the thermoplastic polymer component as a matrix 48, and to force the particles into elongated fibres 46a-c. Specifically, during extrusion, the fibres become embedded in the matrix, and each fibre is forced into having an aspect ratio greater than about fifty to one. The result is a tubing wherein the embedded fibres are all substantially parallel to each other and are oriented substantially parallel to the lumen 42 of the tubing.

## Description

The present invention pertains generally to medical devices and methods for manufacturing same. More particularly, the present invention pertains to medical shafts and tubing which are reinforced with embedded fibres. The present invention is particularly, but not exclusively useful as a medical device, and a method for manufacturing such a device, which incorporates elongated, high aspect ratio fibers made of a low temperature liquid crystal polymer which are used as a reinforcing structure for a low temperature thermoplastic polymer matrix.

Catheters and other similar type medical devices which are used interventionally for the treatment of cardiovascular diseases must have certain desirable physical characteristics in order for them to function properly. Included in these characteristics are flexibility, smoothness, biocompatibility, strength, stiffness and kink resistance. It happens, however, that no one material has an optimal combination of all of these various characteristics. Consequently, it has been necessary to combine different materials in various different ways in order to produce medical devices having acceptably high performance capabilities. In all cases, of course, it is important that the materials which are used are capable of being processed together. Typically, for interventional medical devices such as catheters and tubing, this process involves extrusion.

It happens that many biocompatible materials which have desirable characteristics for flexibility and smoothness, make inherently weak structures. Consequently, without some form of mechanical reinforcement from other materials, these materials are not generally capable of being manoeuvred into and through the cardiovascular system of a patient. Several solutions have been proposed to overcome this problem.

As an example of a multi-material interventional medical device, U.S. Patent No. 5,167,623 which issued to Cianci et al. for an invention entitled "Multilumen Catheter" discloses a device which cooperatively uses different materials having different physical characteristics in different parts of the same product. Yet another example of the combined use of different materials for an interventional device is U.S. Patent No. 5,100,381 which issued to Burns for an invention entitled Angioplasty Catheter. In the Burns device, one material is used as a coating on another material. Additionally it has been proposed that different materials be mixed together to produce a material having the particularly desired characteristics. One such proposal suggests the use of reinforcing fibres which are embedded into the material. For processes which involve extrusion, however, it is nearly impossible to extrude preexisting reinforcing fibres with the polymer materials that are typically used for the manufacture of catheters and other medical tubing. To overcome this difficulty, some attention has been given to the possibility of using liquid crystal polymer (LCP) fibres in extruded polymer medical devices.

U.S. Patent No. 5,156,785 which issued to Zdrahala for an invention entitled "Extruded Tubing and Catheters Having Increased Rotational Stiffness" suggests using liquid crystal polymers (LCPs) as a reinforcement for tubing and catheters that are used as medical devices. The particular LCPs suggested by Zdrahala, however, are of an earlier known type which require processing temperatures in the range above about three hundred and forty-five degrees Centigrade (six hundred and fifty degrees Fahrenheit). These temperatures unfortunately preclude the use of matrix materials which otherwise have very desirable characteristics but which are effectively destroyed at temperatures higher than about two hundred and eighty-five degrees Centigrade (five hundred and fifty degrees Fahrenheit).

In light of the above, it is an object of the present invention to provide a method for manufacturing medical tubing and catheters which uses incompatible polymers that have processing temperatures below approximately two hundred and seventy degrees Centigrade. Another object of the present invention is to provide a method for manufacturing medical tubing and catheters which uses a low-temperature liquid crystal polymer as reinforcing fibres in a matrix to thereby make available a plethora of low-temperature thermoplastic polymers for use as the matrix. Yet another object of the present invention is to provide a tubing or catheter which includes solid state low-temperature reinforcing fibres that give the tubing or catheter enhanced strength, stiffness and kink resistant characteristics. Still another object of the present invention is to provide a method for the manufacture of a medical device which is easy to perform, a tubing or catheter which results from the method that is easy to use, and a resultant product which is comparatively cost effective.

According to a first aspect of the present invention there is provided a shaft defining a longitudinal axis for use in a medical device which comprises a low-temperature thermoplastic polymer matrix having a melt temperature Tₘ and a plurality of solid state low-temperature liquid crystal polymer fibres embedded in said matrix, said liquid crystal polymers having a melt temperature T_{f} with T_{f} being less than twenty degrees Centigrade above Tₘ (T_{f} < Tₘ + 20° C), and said fibres being substantially aligned with said longitudinal axis of said shaft and having an aspect ratio greater than approximately fifty to one (A > 50:1)

According to a second aspect of the present invention there is provided a structure which comprises a plurality of elongate solid state low-temperature liquid crystal polymer fibres aligned substantially parallel to each other, said liquid crystal polymer fibres having a melt temperature T_{f} and said fibres having an aspect ratio greater than fifty to one (A > 50:1) and a low-temperature thermoplastic polymer matrix having a melt temperature Tₘ for maintaining said fibres in said substantially parallel relationship, with Tₘ being within twenty degrees Centigrade of T_{f}.

According to a third aspect of the present invention there is provided a method of manufacturing a shaft for use with a medical device which comprises the steps of blending a low-temperature thermoplastic polymer component having a melt temperature Tₘ with a plurality of discrete low-temperature liquid crystal polymer particles having a melt temperature T_{f} to create a mix; heating said mix to transition both said thermoplastic polymer component and said liquid crystal particles to a substantially liquid phase to create a molten mix; extruding said molten mix to form said shaft, said thermoplastic polymer component being formed as a matrix and said liquid crystal polymer particles being forced into a plurality of discrete elongated fibres, said fibres each having an aspect ratio greater than approximately fifty to one (A > 50:1) and cooling said shaft.

The method includes the step of blending a low-temperature thermoplastic polymer with a plurality of discrete low-temperature liquid crystal polymer particles. This blending creates a mix which is subsequently heated and extruded to manufacture the desired shaft, e.g. a medical device. For the heating and extrusion steps it is important that the low-temperature thermoplastic polymer matrix and the low-temperature liquid crystal polymer be incompatible with each other. In the context of the present invention, the term incompatible is used to mean that the materials, when heated to their processing temperature, do not combine with each other. Stated differently, the liquid crystal polymer remains separated from the thermoplastic polymer matrix.

Preferably, the thermoplastic polymer matrix for the present invention is a material selected from the group consisting of polyamides (nylon), polyurethanes, polyesters and polyolefins. Further, both the thermoplastic polymer material used for the matrix, and the liquid crystal polymer used for the reinforcing fibres should have processing temperatures which are below approximately two hundred and seventy degrees Centigrade (270°C). By having processing temperatures which are below approximately two hundred and seventy degrees Centigrade (270°C), both the thermoplastic polymer matrix and the liquid crystal polymer are identified for the present invention as being "low-temperature". Further, it is preferable that the melt transition temperature of the liquid crystal polymer fibres (T_{f}) be no more than approximately twenty degrees Centigrade above the melt transition temperature (Tₘ) of the thermoplastic polymer material that is used for the matrix. Thus, as an upper value for T_{f} the relationship between T_{f} and Tₘ can be stated as: T_{f} < Tₘ + 20°C. Stated differently, as a lower value for Tm this relationship will be: Tₘ > T_{f} - 20°C.

In the blending step of the method for the present invention, the ratio of thermoplastic polymer to liquid crystal polymer can be varied somewhat according to the particular characteristics that are desired. Therefore, as intended for the present invention, the blending step is accomplished to create a mix with a ratio wherein the thermoplastic polymer is in the range of approximately seventy percent to ninety nine precent by weight and the liquid crystal polymer particles (fibres) are in the range of approximately one percent to thirty percent by weight.

After the blending step, the mixture is heated to transition both the thermoplastic polymer component and the liquid crystal polymer particles into a substantially liquid phase. This creates a molten mix which is extruded in a way well known in the art to form a tubing or catheter. During extrusion, the thermoplastic polymer component is formed as a matrix. Also during extrusion, the liquid crystal polymer particles are forced into a plurality of discrete elongate fibres that are embedded into the matrix. This results from an extensional flow velocity pattern that is created in the extruder cross head adapter and die regions. Preferably, the fibres each have an aspect ratio which is greater than approximately fifty to one.

The result is an interventional medical device in the form of a shaft, tubing, or catheter which is formed as a matrix in which a plurality of fibres are embedded and aligned to give the device enhanced axial strength and kink resistance. Specifically, for a shaft, the fibres will be substantially aligned with the longitudinal axis of the shaft. For a tubing formed with a lumen, the fibres will be substantially aligned with the lumen. Further, for those structures which include a balloon, such as an angioplasty balloon, the fibres will be substantially aligned with the longitudinal axis of the balloon. For purposes of the present invention, the balloon can be formed in any manner well known in the pertinent art.

The novel features of this invention, as well as the invention itself, both as to its structure and its operation, will be best understood from the accompanying description, given by way of example only, taken in conjunction with the accompanying drawings, in which similar reference characters refer to similar parts, and in which:
Figure 1 is a schematic of an extrusion system used for the practice and manufacture of the present invention;
Figure 2 is a perspective view of a shaft and an integral balloon as manufactured by methods according to the present invention, with portions disconnected for clarity; and
Figure 3 is a cross sectional view of a shaft manufactured in accordance with the present invention as seen along the line 3-3 in Figure 2 with the embedded fibres exposed.

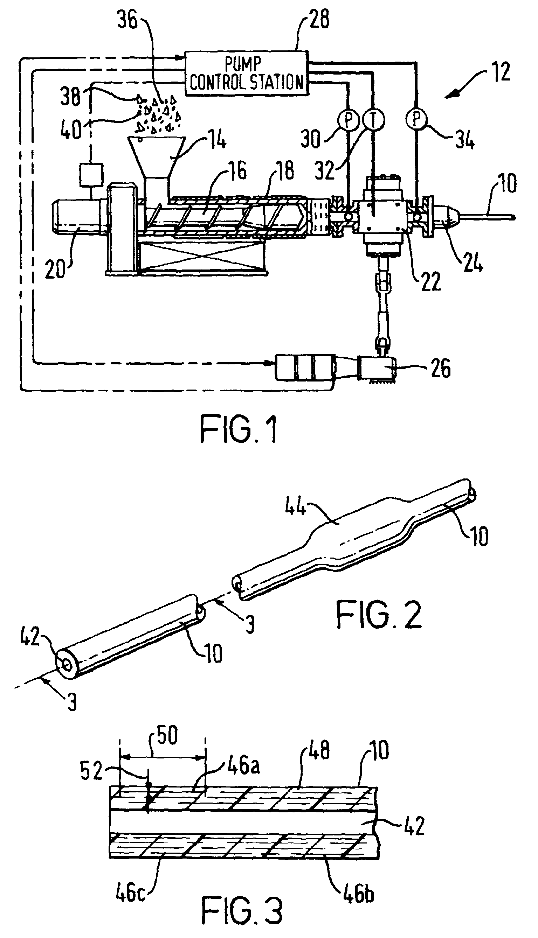

Referring initially to Figure 1, a shaft for an interventional medical device that has been manufactured in accordance with the methods of the present invention is shown and designated 10. Figure 1 shows that the shaft 10 is produced by an extrusion system which is generally designated 12.

Briefly, for purposes of further discussion, as shown in Figure 1 the extrusion system 12 includes a hopper 14 for receiving polymer particles into the system 12. The system 12 also includes an extruder 16 which is directly connected to the hopper 14 for moving the polymer particles as they are being processed. Mounted around the extruder 16 are a plurality of heating elements 18 which melt the polymer particles in the extruder 16 as they are advanced therethrough by the action of the extruder motor 20. As the molten polymer then leaves the extruder 16, it is pressurized by a gear pump 22 and extruder through a die 24 by the action of a drive system 26.

Control over the operation of the extrusion system 12 is provided by a pump control station 28. For precise control, the control station 28 is connected to the gear pump 22 by a series of sensors. These include an inlet pressure sensor 30, a temperature sensor 32, and an outlet pressure sensor 34. Based on the readings and measurements of the sensors 30, 32 and 34, the pump control station 28 controls both the extruder motor 20 and the drive system 26 to form the shaft 10 as desired.

For the methods of the present invention, a mix 36 is first created by blending particles of a thermoplastic polymer 38 with particles of a liquid crystal polymer (LCP) 40. Preferably, an LCP 40 is used which has a low precessing temperature that is in a range between approximately two hundred and twenty, and two hundred and seventy degrees Centigrade (220°C - 270°C). By using such a low temperature LCP 40, it is possible to use a low temperature thermoplastic polymer 38 which also has a low processing temperature. More particularly, in order to avoid destructive effects on the thermoplastic polymer 38 during an extrusion, it is desirable that the highest extrusion processing temperature be not more than about twenty degrees Centigrade above the melt transition temperature of the polymer 38, (Tₘ). Accordingly, it is desirable that an LCP 40 be used which has a melt transition temperature (T_{f}) which is less than approximately twenty degrees Centigrade above Tₘ (i.e. T_{f} < Tₘ + 20°C).

For the present invention, a thermoplastic polymer 38 having an acceptable processing temperature that is usable with an LCP 40 having a T_{f} between 220°C - 270°C can be selected from a group consisting of polyamides (nylon), polyurethanes, polyesters and polyolefins. Examples of melt transition temperatures (Tₘ) for these materials are respectively:
Polyamides (nylon) - Tₘ between two hundred and ten and two hundred and seventy-five degrees Centigrade (210° - 275°C);
Polyurethanes - Tₘ between two hundred and twenty and two hundred and fifty-five degrees Centigrade (220° - 255°C);
Polyesters - Tₘ between two hundred and twelve and two hundred and sixty-five degrees Centigrade (212° - 265°C); and
Polyolefins - Tₘ between two hundred and twenty and two hundred and seventy degrees Centigrade (220° - 270°C)

It is also intended for the present invention that the thermoplastic polymer 38 and the LCP 40 be incompatible with each other. The term "incompatible" as used here means that the two materials do not mix with each other when in their respective molten states.

During the blending of the mix 36 it is possible for the ratio of thermoplastic polymer 38 to LCP 40 to be varied. Depending on the desired results, this variation can be made during the processing of a single batch of mix 36, or from batch to batch. Specifically, it has been determined that a variety of materials having slightly different performance characteristics can be manufactured using different ratios. Preferably, for most medical devices, a ratio is established wherein the thermoplastic polymer 38 creates a matrix which is in the range of approximately seventy percent to ninety nine percent by weight and, respectively, the LCP 40 creates fibres which are in the range of approximately thirty percent to one percent (70:30 - 99:1).

After the mix 36 has been blended, it is introduced into the extrusion system 12 through the hopper 14. As the extruder motor 20 moves the mix 36 through the extruder 16, the heating elements heat the mix 36 to a temperature below approximately two hundred and seventy degrees Centigrade (270°C) to melt the mix 36. Recall the relationship of the respective melt temperatures (T_{f} and Tₘ) for the LCP 40 and the thermoplastic polymer 38. Specifically, as an upper value condition for Tₘ it is desirable for Tm to be within twenty degrees Centigrade of T_{f}. While in the molten state, the thermoplastic polymer 38 and LCPs 40 of the mix 36 are forced through the die 24 by the gear pump 22 to create the shaft 10.

Figure 2 shows that the shaft 10 can be formed with a lumen 42 or with an integral balloon 44. Either of the embodiments shown in Figure 2, or a solid shaft having no lumen 42, are possible for the shaft 10 of the present invention. An important characteristic for all embodiments of the present invention, however, is that the particles of LCP 40 in mix 36 are formed into elongated fibres 46 during the extrusion process.

In Figure 3, a plurality of LCP fibres 46, of which fibres 46a-c are examples, are shown embedded in a matrix 48 that is made from the thermoplastic polymer 38. Using the fibre 46a as an example, it can be appreciated that the aspect ratio for all of the fibres 46 (i.e. the ratio of fibre length 50 to fibre thickness 52) will be greater than approximately fifty to one (50:1). Further, in Figure 3 it will also be appreciated that the fibres 46 are substantially mutually parallel to each other and are aligned substantially parallel to the length of the lumen 42 or to the longitudinal axis of the shaft 10.

## Claims

1. A shaft (10) defining a longitudinal axis for use in a medical device which comprises:
a low-temperature thermoplastic polymer matrix (48) having a melt temperature (Tₘ); and
a plurality of solid state low-temperature liquid crystal polymer fibres (46a-c) embedded in said matrix, said liquid crystal polymers having a melt temperature (T_{f}) with T_{f} being less than twenty degrees Centigrade above Tₘ (T_{f} < Tₘ + 20°C), and said fibres being substantially aligned with said longitudinal axis of said shaft (10) and having an aspect ratio (A) greater than approximately fifty to one (A > 50:1).

2. A structure which comprises:
a plurality of elongate solid state low-temperature liquid crystal polymer fibres (46a-c) aligned substantially parallel to each other, said liquid crystal polymer fibres having a melt temperature (T_{f}) and said fibres having an aspect ratio (A) greater than fifty to one (A > 50:1); and
a low-temperature thermoplastic polymer matrix (48) having a melt temperature (Tₘ) for maintaining said fibers in said substantially parallel relationship, with Tₘ being within twenty degrees Centigrade of T_{f}.

3. A shaft as recited in claim 1 or 2 wherein said low-temperature thermoplastic polymer (38) and said low-temperature liquid crystal polymer (40) are incompatible, wherein said thermoplastic polymer (38) is a material selected from the group consisting of polyamides (nylon), polyurethanes, polyesters and polyolefins having processing temperatures below two hundred and seventy degrees Centigrade (270°C), and wherein said liquid crystal polymer (40) has a processing temperature below two hundred and seventy degrees Centigrade (270°C).

4. A shaft as recited in claim 2 or 3 wherein said thermoplastic polymer matrix and said solid state liquid crystal polymer fibres establish a ratio wherein said thermoplastic polymer matrix is in the range of seventy percent to ninety nine percent by weight and, respectively, said liquid crystal polymer fibres are in the range of thirty percent to one percent by weight (70:30 - 99:1).

5. A schaft as recited in any preceding claim wherein said shaft is a tube (10) and said tube is formed with at least one longitudinal extending lumen (42).

6. A shaft as recited in claim 5 wherein said solid state liquid crystal polymer fibres (46a-c) are substantially aligned with said lumen (42).

7. A shaft as recited in any preceding claim wherein said shaft is a catheter.

8. A shaft as recited in any preceding claim wherein said shaft (10) is formed with an integral balloon (44).

9. A method for manufacturing a shaft (10) for use with a medical device which comprises the steps of:
blending a low-temperature thermoplastic polymer component (38) having a melt temperature (Tₘ) with a plurality of discrete low-temperature liquid crystal polymer particles (40) having a melt temperature (T_{f}) to create a mix;
heating said mix to transition both said thermoplastic polymer component and said liquid crystal polymer particles to a substantially liquid phase to create a molten mix;
extruding said molten mix to form said shaft (10), said thermoplastic polymer component being formed as a matrix (48) and said liquid crystal polymer particles being forced into a plurality of discrete elongated fibres (46a-c), said fibres each having an aspect ration (A) greater than approximately fifty to one (A > 50:1); and
cooling said shaft (10)

10. A method as recited in claim 9 wherein said low-temperature thermoplastic polymer component (38) and said low-temperature liquid crystal polymer (40) are incompatible, wherein said thermoplastic polymer component (38) is a material selected from the group consisting of polyamides (nylon), polyurethanes, polyesters and polyolefins having processing temperatures below two hundred and seventy degrees Centigrade (270°C), and wherein said liquid crystal polymer (40) has a processing temperature below two hundred and seventy degrees Centigrade (270°C).

11. A method as recited in claim 9 or 10 wherein said blending step is accomplished to create a mix wherein said thermoplastic polymer matrix (48) and said solid state liquid crystal polymer fibres (46a-c) establish a ratio wherein said thermoplastic polymer matrix is in the range of seventy percent to ninety nine percent by weight and, respectively, said liquid crystal polymer fibres are in the range of approximately thirty percent to one percent by weight (70:30 - 99:1).

12. A method as recited in any of claims 9 to 11 further comprising the step of forming said shaft as a tube having at least one longitudinally extending lumen(42).

13. A method as recited in claim 12 wherein said solid state liquid crystal polymer fibres are substantially aligned with said lumen (42).

14. A method as recited in claim 12 or 13 further comprising the step of forming an integral balloon (44) in the tube in fluid communication with said lumen (42).

15. A method as recited in any of claims 9 to 14 wherein T_{f} is less than twenty degrees Centigrade above Tₘ (T_{f} < Tₘ + 20°C).

16. A method as recited in any of claims 9 to 14 wherein Tₘ is within twenty degrees Centigrade of T_{f}.
